# FASCICULE DE BREVET EUROPEEN

(11) **EP 2 170 290 B1**
(45) Date de publication et mention de la délivrance du brevet: **21.01.2015**
(21) Numéro de dépôt: 08832081.7
(22) Date de dépôt: 18.07.2008
(51) Int. Cl.: A61K 9/20, A61K 9/70, A61K 47/48

(54) **SYSTEME A LIBERATION CONTROLEE D'UN PRINCIPE ACTIF ET PROCEDE DE PREPARATION**
SYSTEM ZUR GESTEUERTEN FREISETZUNG EINES WIRKSTOFFS SOWIE VERFAHREN ZU SEINER HERSTELLUNG
SYSTEM FOR CONTROLLED RELEASE OF AN ACTIVE PRINCIPLE AND METHOD FOR PREPARATION

(30) Priorité: 18.07.2007 FR 0705187
(43) Date de publication de la demande: 07.04.2010
(73) Titulaire: Centre National de la Recherche Scientifique, 75794 Paris Cedex 16 (FR); Université Montpellier I, F-34000 Montpellier (FR)
(72) Inventeur: VERT, Michel, F-34170 Castelnau-le-lez (FR); LECLERCQ, Laurent, F-34090 Montpellier (FR); BOUSTTA, Mahfoud, F-34570 Pignan (FR)
(74) Mandataire: Desaix, Anne
(86) Numéro de dépôt international: PCT/FR2008/001066
(87) Numéro de publication internationale: WO 2009/037401

(56) Documents cités:
- WO-A-92/11844
- US-A1- 2005 118 718

## Description

La présente invention concerne un système à libération contrôlée d'un principe actif comportant une matrice polymère dégradable ainsi qu'un complexe solide entre ce principe actif et un partenaire polyélectrolyte acido-sensible lui-même dégradable sous l'effet des produits de la dégradation du ou des polymères formant la matrice.

Dans le domaine de la libération contrôlée de principes actifs, et notamment de principes actifs portant des charges électrostatiques multiples, ou encore de polyélectrolytes, tels que peptides, protéines et polynucléotides, il a été proposé de piéger de façon temporaire le principe actif au sein d'une matrice, généralement polymère, de laquelle les molécules de principes actifs relarguent selon un profil conditionné par leur solubilité en fonction d'un partage de phases, de leur aptitude à diffuser au sein de cette matrice ou des défauts qu'elle présente, et/ou de la vitesse de dégradation de celle-ci [3].

Toutefois, l'incorporation des composés macromoléculaires très hydrosolubles, tels que les peptides et les macromolécules de type protéines et oligo- ou polynucléotides, est souvent difficile et peu efficace en raison de leur hydrophilie. En parallèle et pour les mêmes raisons, le relargage est souvent trop rapide en raison de leur grande solubilité dans les fluides biologiques environnants ou d'un gonflement très important de la matrice, comme dans le cas des matrices polymères de type hydrogel.

De plus, le piégeage des principes actifs très hydrophiles, notamment des principes actifs chargés électrostatiquement (anions ou cations) est difficile à réaliser car ces principes actifs sont difficiles à incorporer dans une matrice hydrophobe solide et leur relargage est trop rapide ou trop long selon la nature du système principe actif-matrice.

Enfin, les profils de libération sont jusqu'à présent généralement insatisfaisants, et il existe un besoin pour des systèmes à libération contrôlée dont le profil de libération soit plus adapté. La Demanderesse a rempli ce besoin et atteint ce but pour des principes actifs portant au moins une charge électrostatique, en mettant en oeuvre des matrices polymères, dégradables par dégradation hydrolytique avec génération de composés acides. Contrairement au cas général où le principe actif est directement incorporé dans la matrice, selon l'invention, le principe actif est d'abord engagé, avec un partenaire polyélectrolyte de charge opposée, dans un complexe polyélectrolyte solide. La matrice est dégradée au contact de l'eau ou de liquides physiologiques en générant des acides qui catalysent la dégradation du polyélectrolyte complexant, et le principe actif est libéré.

WO 2006/099514 décrit des systèmes à libération contrôlée où un principe actif d'une part et un complexe polyélectrolyte (CPE) bioactif de l'autre sont ajoutés indépendamment l'un de l'autre à une solution de polymère. Ils sont intégrés à la matrice polymère en solution, par dissolution ou dispersion. Ce document ne décrit ni ne suggère l'utilisation particulière de complexant acido-sensible qui interagirait avec les produits acides de dégradation de la matrice. Dans WO 2006/099514, les CPE, intégrés dans les matrices polymères, modulent la libération d'un principe actif distinct des CPE. Ces CPE sont des solides stables et ne peuvent donc être considérés comme bioactifs. De plus, ils ne peuvent pas diffuser tels quels hors d'une matrice polymère, quelle qu'elle soit. Pour que cela soit possible, il faut que le CPE soit déstabilisé et il est connu que cela est très difficile. Selon l'invention, un des partenaires du complexe est choisi dégradable à cet effet.

La complexation peut notamment masquer l'hydrophilie quand celle-ci est excessive et favoriser l'incorporation du principe actif dans la matrice. L'incorporation du complexe dans une matrice permet de protéger temporairement le principe actif complexé, de freiner ou prolonger sa délivrance et d'éviter la libération initiale brutale.

Selon l'invention, un partenaire polymère de type polyélectrolyte, complexant, dégradable par voie hydrolytique, fixe temporairement un principe actif, sous forme solide, au sein d'une matrice polymère. Au contact de l'eau ou d'un milieu physiologique, le principe actif est libéré progressivement, par dégradation du complexant polyélectrolyte acido-sensible sous l'action des produits de dégradation acides de la matrice.

Ainsi, sont réunies les conditions pour protéger le principe physiologiquement actif ionique ou la substance bioactive polyélectrolytique par complexation avec un polyélectrolyte acido-sensible de charge nette opposée. Cette protection est réalisée sans faire appel à un couplage chimique qui créerait une nouvelle entité, alors qu'une telle entité devrait être approuvée par les instances réglementaires.

Le principe actif ou composé bioactif, mis ainsi sous forme de particules de complexe solide, est rendu insoluble en milieu aqueux, ce qui minimise le relargage initial brutal. Ainsi, de préférence, le complexe est particulaire, solide et stable dans les conditions de pH, de température et de salinité des milieux physiologiques. Selon l'invention, les complexes sont de préférence des précipités ou encore obtenus à l'état solide par lyophilisation ou passage à l'étuve, par exemple.

De plus, selon l'invention, le complexe est temporairement piégé en l'incorporant au sein d'une matrice. Cette seconde protection du principe actif permet une libération retardée conditionnée en grande partie par la vitesse de dégradation du polyélectrolyte et celle de la matrice hydrolytiquement dégradable, sans modification chimique du composé bioactif.

Selon l'invention, les phénomènes mis en jeu sont du type coopératif dans le sens où la complexation intervient dans un domaine très limité de caractéristiques physico-chimiques (pH, pK(s) des fonctions ionisables ou ionisées, température, force ionique, nature des contre-ions et co-ions en présence, densité de charges des composants ioniques, et même souvent ordre d'addition des réactifs). En conséquence, un complexe polyélectrolytique est généralement stoechiométrique en termes de charges électriques opposées et non en termes de poids.

Au contraire, le mélange en termes de poids, tel qu'exploité dans l'art antérieur conduit quasi-inévitablement à des excès de l'un ou de l'autre des composants de signes opposés, l'excès étant alors soumis aux mêmes règles que le composé non complexé et libéré selon des règles de diffusion (ou de dégradation de la matrice).

De plus, la déstabilisation d'un complexe n'obéit pas à la notion de masse, mais à une variation relativement soudaine des conditions extérieures (variation de pH et/ou de force ionique).

Ainsi, il est observé à partir de courbes divulguées dans le document WO 92/11844 qu'il n'y a pas de libération prolongée au-delà de 24 heures, ce qui se traduit par une variation horizontale de la concentration en EPO avec le temps. La seule libération observée est de type brusque (« burst »), et la libération s'arrête à 30% ou 70% selon la composition. Et, à part les différences de relargage de type « burst », les profils de libération BSA-sucrose (neutre) et BSA-protamine (polybase) sont similaires, et on peut en conclure que les mélanges sont faits en poids, et non en charges, et qu'il n'est pas tenu compte des charges électrostatiques qui commandent la complexation.

De plus, dans le document WO 92 /11844, les complexes décrits dans les exemples ne sont pas précipités mais sont en solution, les solutions étant percolées à travers des colonnes de chromatographie.

Le document US 2005/118718 se rapporte à la formation d'un complexe entre un polyaminoacide et une protéine sans que la génération d'acide ou l'acido-sensibilité des polyélectrolytes engagés dans la partie complexe soit décrite.

Ainsi, il est proposé selon l'invention un système à libération contrôlée d'un principe actif comportant au moins (a) une matrice polymère dégradable génératrice de composés acides et (b) au moins un complexe entre un principe actif doté d'au moins une charge électrostatique et un partenaire polyélectrolyte complexant le principe actif, de charge opposée, le complexant étant acido-sensible.

La présente invention concerne également l'utilisation d'un polyélectrolyte acido-sensible comme complexant, dans un système à libération contrôlée d'un principe actif comportant au moins une matrice polymère dégradable génératrice de composés acides et au moins un complexe entre un principe actif doté d'au moins une charge électrostatique et un partenaire polyélectrolyte complexant ledit principe actif, de charge opposée.

Par ailleurs, il est proposé selon l'invention un procédé de préparation d'un système à libération contrôlée comportant au moins les étapes de formation d'un complexe entre un principe actif doté d'au moins une charge électrostatique et un partenaire polyélectrolyte complexant de charge opposée, le complexe étant de préférence sous forme solide et ledit complexant étant acido-sensible, puis l'incorporation du complexe dans une matrice polymère biorésorbable générant des produits de dégradation acides.

Il peut ainsi être formé par appariement ionique un complexe dégradable, stable dans les conditions de pH, de salinité et de température des milieux physiologiques neutres, par exemple sous forme d'un précipité, que l'on peut isoler et incorporer dans une matrice. Un tel appariement ionique suppose une stoechiométrie de charge, par opposition à une stoechiométrie de masse.

Les systèmes à libération contrôlée de l'invention permettent de moduler les caractéristiques de libération du principe actif et ce sans modification chimique du composé bioactif qu'est le principe actif. La modulation est fonction de la stabilité du partenaire polyélectrolyte de charge opposée à celle du principe actif, de la sensibilité du complexe à la dégradation au contact d'acides et de la nature de la matrice, la libération retardée du principe actif étant principalement conditionnée par la vitesse de dégradation du polyélectrolyte dégradable et par la nature de la matrice polymère.

Ces systèmes à libération contrôlée peuvent permettre, en particulier, la libération contrôlée de principes actifs du type oligomères ou de polymères chargés.

La formation du complexe est un élément déterminant de la préparation des systèmes à libération de l'invention. Le complexe est de préférence suffisamment stable dans les conditions mises en oeuvre pour permettre de l'isoler et de l'incorporer dans une matrice. Plus précisément, le complexe est de préférence selon l'invention stable dans des conditions de pH, de salinité et de température typiques du liquide biologique qui est au contact du système de libération. Il s'agit notamment, à pH 7,4 et environ 37°, du sang ou de la lymphe. Ainsi, par milieux physiologiques et dans lesquels le complexe est stable, on entend les milieux physiologiques où le système à libération contrôlé est destiné à être appliqué.

De préférence, on forme un complexe solide obtenu sous forme de poudre, par exemple par précipitation selon l'invention. Selon l'invention, le complexe est ainsi insoluble en milieu aqueux de préparation. On incorpore le complexe dans une matrice polymère dégradable génératrice de composés acides, de façon usuelle pour l'homme de l'art. La granulométrie doit être compatible avec l'incorporation ultérieure, par exemple par enrobage dans le matériau de la matrice.

Le complexe solide peut, dans certains cas, s'il n'est pas obtenu par précipitation, être obtenu par exemple sous forme de lyophilisat. La forme solide du complexe dépend du couple complexant-principe actif, et l'homme de l'art saura choisir et adapter les techniques de formation du complexe en vue de son incorporation dans la matrice.

Comme matrice polymère dégradable, on peut utiliser tout polymère biodégradable susceptible de former des composés acides lors de sa dégradation hydrolytique.

De façon générale, conviennent des matrices du type poly(α-hydroxy acide) ou polyanhydride, et d'autres polymères similaires, physiologiquement acceptables et susceptibles de former des composés acides lors de leur dégradation peuvent être également envisagés, comme certains polyorthoesters. De tels polymères sont par exemple décrits par M. Vert parmi les biopolymères artificiels [8].

A titre d'exemple, on peut citer les matrices poly(acide lactique - co - acide glycolique) (PLAGA). Ce copolymère fournit de l'acide lactique et de l'acide glycolique en se dégradant. On note « PLA » pour poly(acide lactique), et on note couramment PLA50 pour 50% d'unités L-lactique et 50% d'unités D-lactique, PLA(37,5)GA(25) pour 37,5% d'unités L-lactique et 37,5% d'unités D-lactique pour les unités acide lactique et 25% d'unités d'acide glycolique. Plus le taux d'acide lactique est important, plus le caractère hydrophobe de la matrice est marqué et plus la dégradation hydrolytique est lente. On passe typiquement par exemple d'une dégradation en milieu PBS en 18 jours avec PLA(37,5)GA(25) à 90 jours avec l'homopolymère PLA50 [7].

La masse molaire initiale de la matrice est choisie pour permettre de former l'implant ou les particules ou l'hydrogel souhaité selon les techniques usuelles dans le domaine de la libération contrôlée.

On peut citer aussi les matrices poly(caprolactone) (PCL), notamment les PCL modifiées pour être rendues acido-sensibles, telles que celles décrites dans Gimenez et al. [5]. Les polymères PCL fournissent de l'acide caproïque en se dégradant. PCL étant plus hydrophobe que PLA, la dégradation de ce polymère est plus lente que celle de PLA.

On peut aussi citer les dérivés copolymères du poly(β-acide malique) convenablement estérifiés partiellement tels que ceux cités par Mauduit et al. [6].

On peut aussi citer les hydrogels dérivés des polymères précédents, notamment PLA et PLAGA, par combinaison avec des segments hydrophiles.

Comme indiqué ci-dessus, toute autre matrice capable de fournir des produits de dégradation acides et physiologiquement acceptables est envisageable.

La matrice polymère dégradable peut être utilisée sous toute forme usuelle, comme des implants, des films, des microparticules, des hydrogels et notamment des matrices hydrophiles à base de copolymères comportant des segments hydrophiles tels que poly(vinyl pyrrolidone), dextrane ou poly(oxyde d'éthylène) et des segments de polymères générant des composés acides tels que les polyanhydrides ou les poly(α-hydroxy-acides), la forme choisie devant permettre l'incorporation du complexe acido-sensible.

Par principe actif ou composé bioactif ionique susceptible d'être libéré selon la présente invention, on entend tout principe actif ou plus largement tout composé bioactif ionique doté d'au moins une charge électrostatique, ou encore toute substance à caractère polyélectrolytique, c'est-à-dire possédant au minimum une charge électrostatique.

Le procédé de l'invention est particulièrement intéressant pour permettre la libération contrôlée de protéines, gènes et fragments d'ADN, oligonucléotides, par exemple, y compris les petits peptides.

Le composé bioactif ionique est associé à un partenaire polyélectrolyte le complexant, doté d'une charge opposée. De préférence, le composé bioactif ionique est lui-même un polyélectrolyte. Tout principe actif ionique ou oligomère chargé pouvant former une combinaison solide de type complexe ionique ou polyionique peut être utilisé.

Le partenaire polyélectrolyte doit être acido-sensible et porteur de charges opposées de façon à pouvoir former un complexe de type polysel ou complexe polyélectrolytique solide et stable dans les conditions de mise en oeuvre.

Comme partenaire polyélectrolytique selon l'invention, on entend tout polymère acido-sensible physiologiquement acceptable capable de complexer le principe actif.

Selon l'invention, un complexant est considéré comme étant acido-sensible lorsqu'il est stable à un pH de 7,4 et qu'il se dégrade à un pH inférieur à 7. Ainsi, est considéré acidosensible selon l'invention un complexant dégradable en milieu acide.

La dégradation est d'autant plus rapide que le pH du milieu environnant est acide.

Par exemple, les complexants PMLA, PLCA, et PSA se dégradent de manière significative lorsqu'ils sont en contact avec un pH inférieur à 6. Par exemple, un tel pH règne dans les vacuoles lysosomales intracellulaires.

De préférence, on utilise des polyélectrolytes dégradables et biorésorbables, c'est-à-dire éliminables par voie pulmonaire et/ou rénale. Ainsi, avantageusement, le polyélectrolyte acido-sensible complexant appartient à la famille des polymères dits « biopolymères artificiels » [8]. De tels polymères ou copolymères, dégradables, biorésorbables et biocompatibles sont composés d'unités de répétition issues de monomères normalement présents dans les circuits biochimiques et régénérés lors de la dégradation hydrolytique, et enchaînées les unes aux autres par des fonctions chimiques intrinsèquement acido-sensibles telles que les fonctions ester, en particulier ester aliphatique, les fonctions anhydrides, les fonctions orthoester, ou encore des fonctions réputées stables en milieu aqueux, telles que les fonctions amide ou oside, lorsqu'elles sont rendues acido-sensibles par la présence d'autres fonctions promotrices dans leurs formules.

L'archétype de tels biopolymères artificiels de type polyester est un polymère comportant des liaisons intrachaîne [-COO-]ₙ et un reste d'unités de répétition comportant au moins une fonctions ionisable telle que acide carboxylique (exemple : PMLA) ou basique (exemple : PSA). L'archétype des polymères rendus acido-sensibles par la présence d'une autre fonction est le PLCA, la fonction COOH rendant le polymère hydrosoluble et sensibilisant les fonctions amide [- CONH -]ₙ intrachaîne aux acides et donc aux milieux acides, alors que ces dernières sont normalement résistantes que ce soit dans les polymères de type polyamide aliphatique ou aromatique de même que dans les protéines, ces dernières nécessitant l'intervention d'enzymes.

Par exemple, les polymères de type PMLA et PLCA peuvent être utilisés pour complexer tout principe actif ou substance bioactive ou oligomère de charge nette positive. Les polymères de type PSA peuvent être utilisés pour complexer tout principe actif ou substance bioactive ou oligomère de charge nette négative. Les polymères PMLA, PLCA, PLCAI et PSA sont des polyélectrolytes acido-sensibles capables de complexer non seulement des oligomères ou des polymères de charge opposée mais aussi des composés d'intérêt de type acide ou basique tels que des peptides ou des fragments d'ADN. Plus précisément, les polymères PSA, PMLA et PLCA sont définis par les unités suivantes (1) à (4), les unités PLCA étant issues par hydrolyse des unités de l'imide PLCAI (3), représentées au tableau I ci-après. Les unités PLCA (4) sont des combinaisons d'unités lysine et d'unités d'acide citrique. PMLA et PLCA sont connus notamment par US 4 265 247 et EP 332 530 et PSA est décrit par Rossignol, H., et al. [4]. Voir aussi [1], [2] et [8].

De façon préférée, on utilise selon l'invention des polymères PMLA, PLCA, PLCAI et PSA représentés au tableau I ci-après. D'autres contre-ions différents de Na⁺ et Br⁻ peuvent être utilisés. De même, les formes - COOH pour les polyacides et -NH₂ pour les polybases sont envisageables.

Pour les complexants comme pour les matrices polymères, des masses Mw de l'ordre de 5000 à 500 000 g/mol sont envisageables, de préférence de l'ordre de 10 000 à 100 000 g/mol.

Comme exemples de composé bioactif ionique selon l'invention, on peut citer des peptides ou des protéines : bursine, leuprolide, triptoréline, autres analogues de LH-RH, fragments de protéine, inhibiteurs de protéines kinase, etc... Parmi les principes actifs basiques, on peut citer également les composés basiques du type multiamine autres que peptides ou protéines.

**Tableau I**

| | |
|---|---|
| 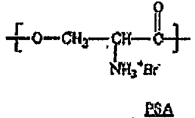 | (1) -[-O-CH₂-CH(NH₃⁺Br⁻)-CO-]- |
| 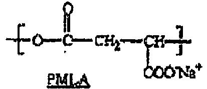 | (2) -[-O-CO-CH₂-CH(COO⁻Na⁺)-]- |
| 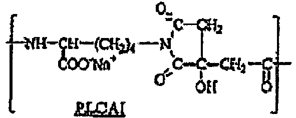 | (3) |
| 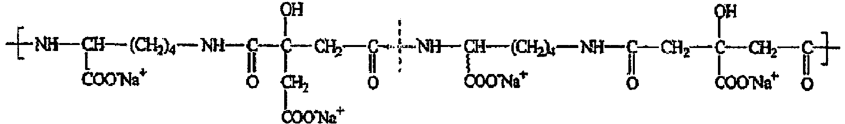 | |
| (4) -[-NH-CH(COO⁻Na⁺)-(CH₂)₄-NH-CO-C(OH)(CH²-COO⁻Na⁺)-CH₂-CO-:-NH- -CH(-COO⁻Na⁺)-(CH₂)₄-NH-CO-CH₂-C(OH)(COO⁻Na⁺)-CH₂-CO-]- | |

En ce qui concerne le procédé de préparation des systèmes de libération de l'invention, de façon générale, les complexes sont formés par mélange d'une solution du polyélectrolyte avec le composé principe actif à protéger et libérer progressivement. De préférence, la complexation conduit à un précipité stoechiométrique pulvérulent. Celui-ci peut être broyé, tamisé ultérieurement afin de faciliter l'incorporation dans la matrice.

On peut lyophiliser le complexe, afin d'en obtenir une forme sèche ; on peut aussi envisager de l'étuver.

L'incorporation du complexe dans la matrice, menant au système ternaire matrice-complexant-principe actif, est réalisée par tout moyen compatible avec la nature des complexes et des matrices. On peut utiliser une matrice sous forme flexible et, pour plier le film, travailler à une température supérieure à Tg.

Typiquement, on utilise une matrice sous forme d'un film, parfois appelé « plaque » sur lequel le complexe pulvérulent est déposé, puis on replie le film sur lui-même et on presse à nouveau le film replié pour obtenir une nouvelle « plaque » dans laquelle le principe actif est dispersé. On peut pour cela utiliser une presse usuelle capable de travailler à température variable et produire des films de quelques centaines de microns d'épaisseur.

Après incorporation du polyélectrolyte à la matrice, la dégradation du polyélectrolyte complexant dégradable au sein de la matrice génératrice d'acides est conditionnée par la présence d'eau ou de liquide physiologique.

La présente invention fait état de combinaisons entre complexes de composés de charges opposées stables dans les conditions physiologiques et de piégeage dans une matrice génératrice d'acides capables de dégrader le complexant polyélectrolyte du principe actif.

Le complexe stable ne peut pas être dissocié par le biais de la loi d'action de masses mais seulement par variation du pH local (obtenue par la dégradation de la matrice) ou encore par augmentation de la force ionique (ce qui n'est pas exploitable à l'intérieur d'une matrice polymère) si bien que le relargage brusque (burst) est atténué, voire supprimé selon l'invention.

Enfin, selon l'invention, les relargages des composés d'intérêt sont réellement prolongés selon des profils originaux, du fait de la complexation avec le polyélectrolyte acido-sensible, la concentration dans la phase de libération évoluant avec le temps sur des périodes longues (plusieurs dizaines de jours selon les cas).

En ce qui concerne les rapports pondéraux, le complexe peut être incorporé dans une large gamme de valeurs du taux pondéral de complexe dans le système ternaire, allant de 0,1 à 60% en poids du poids total du système ternaire. Il est préférable d'exploiter une gamme plus restreinte compatible avec les résultats de pharmacocinétique, une telle gamme préférée va de 1 à 40% en poids du complexe par rapport au poids total du système ternaire complexe-matrice polymère.

Un des avantages du système à libération contrôlée est sa stabilité lors de son stockage. En effet, la dégradation du polymère, par exemple du polyanion complexant dégradable au sein de la matrice génératrice d'acides étant conditionnée par la présence d'eau, le système reste stable lors du stockage à sec.

Alternativement, on peut prévoir de mettre en oeuvre le système ternaire matrice/complexe polyélectrolyte-principe actif non pas sous forme de film mais sous forme de particules. Ces particules pourront être également enrobées classiquement par des polymères de type PLAGA ou PLA afin d'apporter une modulation supplémentaire dans la vitesse de libération du principe actif. La forme de la matrice (microsphères, microparticules, plaques, implants, films, ...) influe sur la vitesse de dégradation de ladite matrice et donc de la vitesse de libération du principe actif. La forme et les dimensions des systèmes à libération contrôlées permettent d'adapter la vitesse de dégradation de la matrice et donc de la formation des produits acides dégradant le partenaire polyélectrolyte.

L'invention sera mieux comprise au vu des figures annexées et des exemples qui suivront.

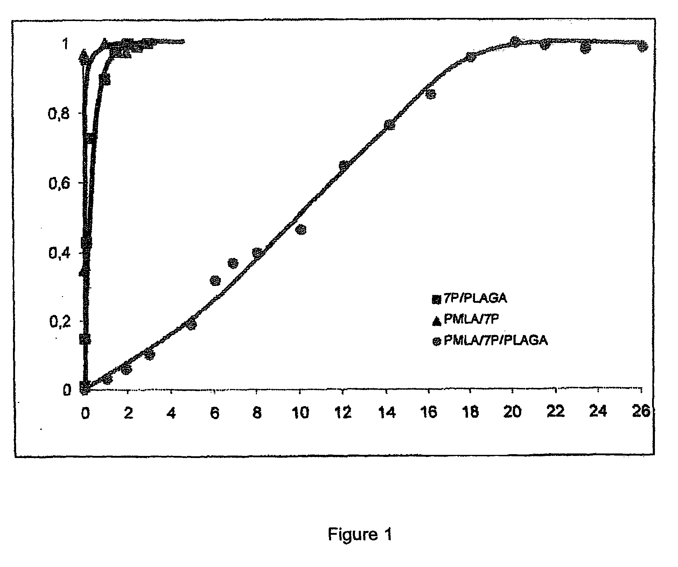

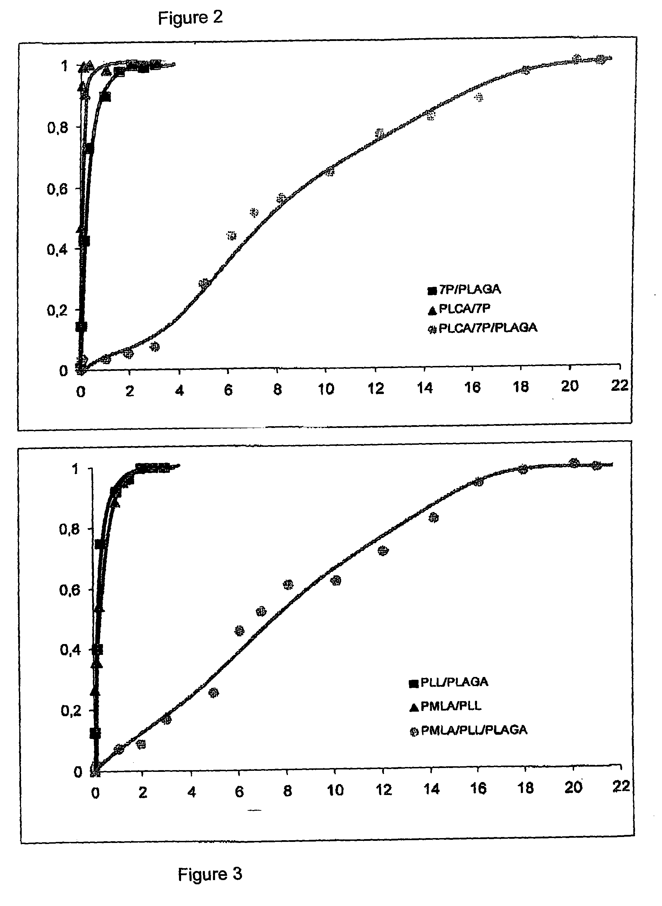

La figure 1 représente le profil de libération d'un principe actif (7P) incorporé dans un complexe de l'invention (complexant PMLA) au sein d'une matrice (PLAGA) (●), par comparaison avec le complexe seul (▲) ou avec le principe actif seul (■), incorporés dans la même matrice. Sur cette figure 1, la dégradation en jours est portée en abscisse, et le taux de libération est porté en ordonnée. Les figures 2 et 3 concernent des exemples similaires avec un autre complexant (PLCA) et un modèle de principe actif polyélectrolytique (PLL) respectivement.

La demanderesse a mis en évidence, comme cela apparaît dans les exemples, que des résultats similaires peuvent être obtenus avec différents principes actifs pour un même couple matrice-complexant en accord avec un contrôle par la dégradation de la matrice, et donc par la cinétique d'apparition de résidus acides.

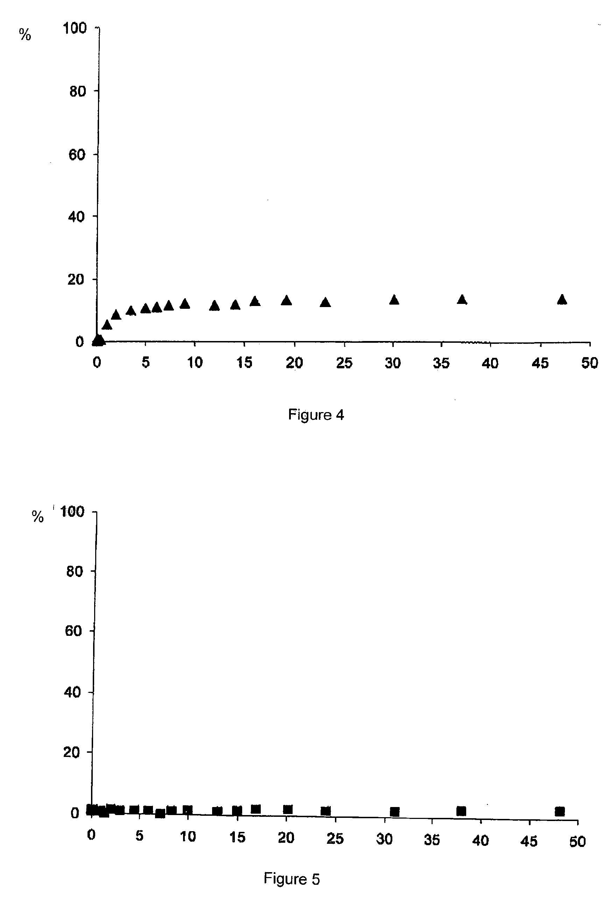

A titre comparatif, les figures 4 et 5 illustrent les profils de libération (pourcentage de libération en ordonnée, en fonction du temps en abscisse, en jours : 0 à 50 jours) dans le cas où un complexant acido-sensible est incorporé dans une matrice stable et le cas où un complexant non acido-sensible est incorporé dans une matrice dégradable.

Les résultats obtenus dans les exemples comparatifs dans le cas d'une matrice stable associée à un complexant non acido-sensible et celui d'une matrice dégradable associée à un complexant acido-sensible montrent respectivement l'importance du choix d'une matrice hydrolytiquement dégradable génératrice de produits de dégradation acides, et l'importance du choix d'un complexant acido-sensible qui se dégrade sous l'effet de la dégradation de la matrice pour permettre la libération prolongée du principe actif.

### Exemples 1 à 5: Préparation de systèmes à libération contrôlée de Arg-Lys-Arg-Ser-Arg-Lys-Glu (7P) et de polylysine (PLL).

### 1°) Réactifs utilisés :

Complexant :
   - PMLA,Na (Mw=30000 g/mol)
   - PLCA,Na (Mw=40000 g/mol, Mn=20000 g/mol)
Matrice :
   - PLA(37,5)GA(25) (Mw=30000 g/mol, Mn=9000 g/mol) pour les exemples 1 à 4
   - PLA50 (Mw=50000 g/mol) pour l'exemple 5
Principe actif ou polyélectrolyte-modèle :
   - L'heptapeptide (7P) ci-dessus, inhibiteur de protéine kinase, a une masse moléculaire de 959 g/mol et contient 5 charges positives globales.
   - La polylysine (PLL) a une masse moléculaire Mw de 12 000 g/mol et contient une charge positive par motif.

7P et PLL sont disponibles notamment auprès des fournisseurs Sigma-Aldrich, Bachem et American Peptide Company.

### 2°) Préparation de Quatre complexes

Ex 1 : Typiquement, 500 µl de solution aqueuse contenant 25 mg de PLL,HBr ont été ajoutés à 1 ml de solution aqueuse contenant 16,7 mg de PMLA,Na. 26 mg de complexe solide PMLA-PLL (rendement = 88%) ont été récupérés après centrifugation du mélange et séchage du précipité.
Ex 2 : De la même manière, 28 mg de PLCA-PLL (rendement = 84%) ont été obtenus à partir de 20,7 mg de PLCA,Na et de 25 mg de PLL, HBr.
Ex 3 : De même 22 ,3 mg de complexe solide PLCA-7P (rendement = 63%) ont été obtenus à partir de 19,1 mg de PLCA,Na et 25 mg de 7P.
Ex 4 : De même, 23,7 mg de complexe solide PMLA-7P (rendement = 75%) ont été obtenus à partir de 15,3 mg de PMLA,Na et 25 mg de 7P.

Les complexes solides ont ensuite été broyés au mortier et une poudre a été obtenue.

### 3°) Incorporation des complexes dans la matrice

Typiquement, pour les systèmes des exemples 1 à 4, 1 g de PLA(37,5)GA(25) a été pressé dans une presse (CARVER 4120 CE) à 50°C et un film de quelques centaines de microns d'épaisseur a été obtenu.

La poudre de complexe a été déposée autour du centre du film, qui a été replié avant de presser de nouveau le film dans les mêmes conditions. Les étapes de pliage et pressage sont répétées trois fois. La totalité du complexe a ainsi été répartie de manière homogène au sein de la matrice.

Pour l'exemple comparatif où le principe actif est incorporé seul, dans la matrice, l'incorporation est réalisée dans les mêmes conditions que celles du complexe.

On a réalisé l'incorporation à un taux pondéral d'incorporation du complexe dans la matrice de 4% en poids, avec une épaisseur finale de la matrice de 0,5 mm, pour les systèmes des exemples 1 à 4.

De plus, avec le même procédé, quand 1 g de PLA50 a remplacé 1 g de PLAGA, le pressage a lieu à 100°C et un film de quelques centaines de microns d'épaisseur a été obtenu (exemple 5).

### Exemples 6 à 8 : Libération du principe actif ionique

Pour chacun des complexes des exemples 1 à 4, on a étudié les trois systèmes suivants : le système binaire complexe solide polyélectrolyte/principe actif (▲ sur les courbes), le système binaire matrice/principe actif (■ sur les courbes), et le système ternaire matrice/complexe solide polyélectrolyte-principe actif (● sur les courbes).

Chacun des trois systèmes a été introduit dans des piluliers contenant 5 ml de PBS (conditions physiologiques modèles) et placés sous agitation à 37°C.

Régulièrement, 20 µl de solution sont prélevés pour analyse et remplacés par un même volume de PBS. Les 20 µl de solution prélevés sont ajoutés à une solution contenant 1,2 ml de tampon borate (concentration 0,1 M, pH=9,3). 20 µl de TNBS (acide trinitrobenzènesulfonique, de concentration 0,03 M) sont ensuite ajoutés au mélange laissé au repos pendant 2 heures. La quantité de groupes amines (et donc de principe actif) libérée est mesurée par spectroscopie UV-Visible à 420 nm au moyen de courbes d'étalonnage.

### Profils de libération - Résultats

La dégradation de la matrice fournit des produits acides qui catalysent la dégradation du complexe polyélectrolyte-principe actif en dégradant de manière sélective le partenaire polyélectrolyte. La vitesse de dégradation de la matrice conditionne directement la vitesse de libération de la substance ionique bioactive.

A la figure 1, dans le cas des systèmes 7P/PLAGA (■) et PMLA/7P (▲), le peptide 7P est libéré en moins de 2 jours, voire en quelques heures pour le système binaire PMLA/7P (▲).

Il est apparu que le système ternaire PMLA/7P/PLA(37,5)GA(25) (●) de la figure 1, comme les systèmes PMLA/PLL/PLA(37,5)GA(25) (●) de la figure 3, PLCA/7P/PLA(37,5)GA(25) (●) de la figure 2 et PLCA/PLL/PLA(37,5)GA(25) (non représenté) permettent un gain de libération de PLL et 7P prolongée sur 18 jours par rapport aux systèmes binaires matrice/principe actif ou complexe polyélectrolyte-principe actif.

Il apparaît par ailleurs que le système binaire PLCA-PLL ne permet pas la libération de PLL dans les conditions physiologiques modèles utilisées : le système ternaire a cependant l'avantage de rendre PLL biodisponible.

En utilisant PLA50 à la place de PLAGA comme matrice dégradable dans le cas du PLL incorporé selon l'exemple 5, le relargage du principe actif est considérablement retardé et s'effectue après trois mois.

### Exemple 9

On a également étudié le système ternaire PLA50/PMLA/7P préparé dans les mêmes conditions que celles de l'exemple 5. Il montre également un relargage au bout de 3 mois.

### Exemples 10 à 12

Des profils de libération similaires ont été obtenus avec des systèmes similaires à ceux des exemples 1, 3 et 4, le taux pondéral étant porté de 4 % à un taux de 8 % dans la matrice. La matrice obtenue finalement a une épaisseur 1,3 mm.

### Exemples 13 - 14

On a préparé des complexes précipités stables dans PBS de façon similaire à celle des exemples 1 et 2, avec le leuprolide à la phase de PLL complexé d'une part avec PMLA, d'autre part avec PLCA.

### Exemples 15 - 16

On a également préparé des complexes PMLA-Bursine et PLCA-Bursine, non pas précipités comme dans les exemples 1 à 4 mais lyophilisés, les lyophilisats étant incorporés dans une plaque de PLAGA.

### Exemples 17 (comparatif)

Avec le complexant PLCA,Na et le principe actif (polyélectrolyte-modèle) PLL, on a préparé le complexe PLCA-PLL et on l'a incorporé dans la matrice la matrice poly(ε-caprolactone) (PCL), comme précédemment à l'exemple 2. L'incorporation a été réalisée à un taux pondéral d'incorporation du complexe dans la matrice de 4% en poids, avec une épaisseur finale de la matrice de 0,5 mm après pressage à 70°C.

Comme cela apparaît à la figure 4, un léger burst attribuable au lavage de surface apparaît. Il correspond à 10% de la quantité totale de PLL. Même après 50 jours, moins de 15% de la PLL totale sont libérés.

### Exemple 18 (comparatif)

Avec le complexant non acido-sensible poly(acide méthacrylique) (PMA,Na) et le principe actif (polyélectrolyte-modèle) PLL, on a préparé le complexe PMA-PLL en ajoutant 500 µl de solution aqueuse contenant 25 mg de PLL,HBr à 1 ml de solution aqueuse contenant 12,9 mg de PMA,Na. 18 mg de complexe solide PMA-PLL (rendement=71%) ont été récupérés, après centrifugation du mélange et séchage du précipité. Le complexe PMA-PLL a été incorporé dans la matrice PLA(37,5)GA(25), comme précédemment aux exemples 1 à 4. L'incorporation a été réalisée à un taux pondéral d'incorporation du complexe de la matrice de 5% en poids, avec une épaisseur finale de la matrice de 0,15 mm après pressage à 60°C.

Comme cela apparaît à la figure 5, même après 50 jours, moins de 3% de la PLL totale sont libérés.

### Exemple 19

Avec la poly(α-aminosérinate) (PSA) et le principe actif (polyélectrolyte-modèle) polyacide acrylique (PAA, Na), on a préparé le complexe PAA-PSA ajoutant 500 µl de solution aqueuse contenant 10,5 mg de PSA,HBr à 1 ml de solution aqueuse contenant 5,5 mg de PAA,Na. 6,8 mg de complexe solide PAA-PSA (rendement=68%) ont été récupérés après centrifugation du mélange et séchage du précipité). Le complexe PAA-PSA obtenu a été incorporé de la même façon qu'aux exemples 1 à 4 dans une matrice PLA (37,5)GA(25). L'incorporation a été réalisée à un taux pondéral d'incorporation du complexe dans la matrice de 4,5% en poids, avec une épaisseur finale de la matrice de 0,22 mm après pressage à 60°C.

Après trois semaines, on a constaté que le profil de libération du PAA (matrice dégradable + complexant polycationique acido-sensible) est analogue à celui d'un complexant polyanionique acido-sensible et d'un principe actif ou polyélectrolyte-modèle de type polycationique des exemples 6 à 8.

### REFERENCES

[1] Release of the polyanion from polyelectrolyte complexes by selective dégradation of the polycation T. Etrych, M. Boustta, L. Leclercq et M. Vert - J. Bioact. Comp. Polym., Vol. 21, pp. 89-105 (2006).
[2] Degradable polymers as tools for polyelectrolyte complex analysis, L. Leclercq, M. Boustta et M. Vert - ACS Symposium Series 939 "Degradable polymers and materials" - Editeurs K.C. Khemani and C. Scholz, Chapter 17, pp. 267-281 (2006).
[3] Influence of the poly(lactide-co-glycolide) type on the leuprolide release from in situ forming microparticle systems X. Luan et R. Bodmeier - J. Controlled Rel., Vol. 110, pp. 266-272 (2006).
[4] Synthetic poly(β-hydroxyalkanoates) with carboxylic acid or primary amine pendent groups and their complexes, Rossignol, H., Boustta, M. & Vert, M., International Journal of Biological Macromolecules 25, 255-264 (1999).
[5] Synthesis, properties and in vitro degradation of carboxyl-bearing PCL, Gimenez, S., Ponsart, S., Coudane, J. & Vert, M., Journal of Bioactive and Compatible Polymers 16, 32-46 (2001).
[6] Hydrolytic Degradation of benzylated poly(beta-malic acid), Mauduit, J.; Boustta, M. et Vert, M., Journal Biomaterials Science, Polymer Edition 7, 207-220 (1995).
[7] Polylactic and polyglycolic acids as drug delivery carrier L. Brannon-Pepas & M. Vert (2000) Handbook of Pharmaceutical Controlled Release Technology, Editeurs : D.L. Wise, A. Kilbanov, R. Langer, A. Mikos, L. Brannon-Pepas, N.A. Peppas, D.J. Trantalo, G.E. Wnek & M.J. Yaszemski, Marcel Dekker, New-York, pp 99-130.
[8] Biopolymers and artificial biopolymers in biomedical applications, an overview. Vert, M., in Biorelated Polymers : Sustainable Polymer Science and Technology. Editeurs : Chiellini et al., Kluwer Academic /Plenum Publishers, (2001).

## Revendications

1. Système à libération contrôlée d'un principe actif comportant au moins (a) une matrice polymère hydrolytiquement dégradable génératrice de composés acides et (b) au moins un complexe entre un principe actif doté d'au moins une charge électrostatique et un partenaire polyélectrolyte complexant le principe actif, de charge opposée, ledit complexant étant acido-sensible stable à un pH de 7,4 et dégradable à un pH inférieur à 7, le complexe solide étant formé par appariement stoechiométrique de charges.

2. Système selon la revendication 1, **caractérisé en ce que** la matrice polymère est du type poly(α-hydroxy acide) ou polyanhydride ou polycaprolactone.

3. Système selon la revendication 1 ou 2, **caractérisé en ce que** la matrice polymère est du type poly(lactide-co-glycolide).

4. Système selon l'une des revendications 1 à 3, **caractérisé en ce que** la matrice polymère dégradable est à base de copolymères comportant des segments hydrophiles tels que poly(vinyl pyrrolidone), dextrane ou poly(oxyde d'éthylène) et des segments de polymères générant des composés acides tels que les polyanhydrides ou les poly(α-hydroxy-acides).

5. Système selon l'une des revendications 1 à 4, **caractérisé en ce que** le complexant est choisi parmi les complexants polyélectrolytes du type poly(acide malique) (PMLA), poly(lysine citramide) (PLCA ou PLCAI) et poly(amino sérinate) (PSA).

6. Système selon l'une des revendications 1 à 5, **caractérisé en ce que** le complexe est constitué par appariement ionique entre un principe actif basique et un complexant polyanion du type poly(acide malique) ou poly(lysine citramide).

7. Système selon l'un des revendications 1 à 6, **caractérisé en ce que** le principe actif basique est du type multiamine.

8. Système selon l'une des revendications 1 à 5, **caractérisé en ce que** le complexe acido-sensible est constitué par appariement ionique entre un principe actif acide et un complexant polycation acido-sensible du type poly(amino sérinate) (PSA).

9. Système selon l'une des revendications 1 à 8, **caractérisé en ce que** le complexe est insoluble en milieu aqueux.

10. Système selon l'une des revendications 1 à 9, **caractérisé en ce que** le principe actif est un polyélectrolyte.

11. Système selon l'une des revendications 1 à 10, **caractérisé en ce que** le taux de complexe dans le système est dans la gamme allant de 0,1 à 60 % en poids, de préférence 1 à 40 % en poids.

12. Procédé de préparation d'un système à libération contrôlée selon l'une des revendications 1 à 11 comportant au moins les étapes de formation d'un complexe entre un principe actif doté d'au moins une charge électrostatique et un partenaire polyélectrolyte complexant de charge opposée, acido-sensible stable à un pH de 7,4 et dégradable à un pH inférieur à 7, ledit complexe étant stable dans des conditions de pH, de salinité et de température des milieux physiologiques, puis l'incorporation dudit complexe dans une matrice polymère biorésorbable générant des produits de dégradation hydrolytique acides.

13. Utilisation d'un polyélectrolyte acido-sensible comme complexant, dans un système à libération contrôlée d'un principe actif comportant au moins une matrice polymère dégradable génératrice de composés acides et au moins un complexe entre un principe actif doté d'au moins une charge électrostatique et un partenaire polyélectrolyte complexant ledit principe actif, de charge opposée, le complexe solide étant formé par appariement stoechiométrique de charges, le polyélectrolyte étant stable à un de pH 7,4 et dégradable à un pH inférieur à 7.

14. Utilisation selon la revendication 13, **caractérisée en ce que** la matrice polymère est du type poly(α-hydroxy acide) ou polyanhydride ou polycaprolactone, de préférence, PLA ou PLAGA, ou est à base de copolymères comportant des segments hydrophiles tels que poly(vinyl pyrrolidone), dextrane ou poly(oxyde d'éthylène) et des segments de polymères générant des composés acides tels que les polyanhydrides ou les poly(α-hydroxy-acides).

15. Utilisation selon la revendication 13 ou 14, **caractérisée en ce que** le complexant est choisi parmi PMLA, PLCA, PLCAI ou PSA.

## Patentansprüche

1. System zur gesteuerten Freisetzung eines Wirkstoffs mit wenigstens (a) einer saure Verbindungen erzeugenden, hydrolytisch abbaubaren Polymermatrix und (b) wenigstens einer Komplexverbindung zwischen einem mit wenigstens einer elektrostatischen Ladung versehenen Wirkstoff und einem den Wirkstoff komplexierenden Polyelektrolytpartner mit entgegengesetzter Ladung, wobei der Komplexbildner bei einem pH-Wert von 7,4 säureempfindlich stabil und bei einem pH-Wert unter 7 abbaubar ist, wobei der feste Komplexbildner durch stöchiometrische Ladungspaarung gebildet wird.

2. System nach Anspruch 1,
**dadurch gekennzeichnet, dass** die Polymermatrix vom Typ Poly-α-Hydroxysäure oder Polyanhydrid oder Polycaprolacton ist.

3. System nach Anspruch 1 oder 2,
**dadurch gekennzeichnet, dass** die Polymermatrix vom Typ Polylactid-co-Glycolid ist.

4. System nach einem der Ansprüche 1 bis 3,
**dadurch gekennzeichnet, dass** die abbaubare Polymermatrix auf Copolymeren mit hydrophilen Segmenten, wie Polyvinylpyrrolidon, Dextran oder Polyethylenoxid und saure Verbindungen erzeugenden Polymersegmenten, wie Polyanhydride oder Poly-α-Hydroxysäuren basiert.

5. System nach einem der Ansprüche 1 bis 4,
**dadurch gekennzeichnet, dass** der Komplexbildner ausgewählt ist aus Polyelektrolyt-Komplexbildnern vom Typ Polyapfelsäure (PMLA), Polylysincitramid (PLCA oder PLCAI) und Polyaminoserinat (PSA).

6. System nach einem der Ansprüche 1 bis 5,
**dadurch gekennzeichnet, dass** die Komplexverbindung durch Ionenpaarung zwischen einem basischen Wirkstoff und einem Polyanion-Komplexbildner vom Typ Polyapfelsäure oder Polylysincitramid gebildet ist.

7. System nach einem der Ansprüche 1 bis 6,
**dadurch gekennzeichnet, dass** der basische Wirkstoff vom Typ Multiamin ist.

8. System nach einem der Ansprüche 1 bis 5,
**dadurch gekennzeichnet, dass** die säureempfindliche Komplexverbindung durch Ionenpaarung zwischen einem sauren Wirkstoff und einem säureempfinlichen Polykation-Komplexbildner vom Typ Polyaminoserinat (PSA) gebildet ist.

9. System nach einem der Ansprüche 1 bis 8,
**dadurch gekennzeichnet, dass** die Komplexverbindung in wässrigem Medium unlöslich ist.

10. System nach einem der Ansprüche 1 bis 9,
**dadurch gekennzeichnet, dass** der Wirkstoff ein Polyelektrolyt ist.

11. System nach einem der Ansprüche 1 bis 10,
**dadurch gekennzeichnet, dass** der Anteil der Komplexverbindung in dem System zwischen 0,1 und 60 Gew.-%, vorzugsweise zwischen 1 und 40 Gew.-% liegt.

12. Verfahren zur Herstellung eines Systems zur gesteuerten Freisetzung nach einem der Ansprüche 1 bis 11, umfassend wenigstens die Schritte: Bildung einer Komplexverbindung zwischen einem mit wenigstens einer elektrostatischen Ladung versehenen Wirkstoff und einem komplexierenden Polyelektrolytpartner mit entgegengesetzter Ladung, der bei einem pH-Wert von 7,4 säureempfindlich stabil und bei einem pH-Wert von unter 7 abbaubar ist, wobei die Komplexverbindung unter pH-, Salzgehalts- und Temperaturbedingungen der physiologischen Medien stabil ist, dann Einbettung der Komplexverbindung in eine bioresorbierbare Polymermatrix, die saure hydrolytische Abbauprodukte erzeugt.

13. Verwendung eines säureempfindlichen Polyelektrolyts als Komplexbildner in einem System zur gesteuerten Freisetzung eines Wirkstoffs mit wenigstens einer saure Verbindungen erzeugenden abbaubaren Polymermatrix und wenigstens einer Komplexverbindung zwischen einem mit wenigstens einer elektrostatischen Ladung versehenen Wirkstoff und einem diesen Wirkstoff komplexierenden Polyelektrolytpartner mit entgegengesetzter Ladung, wobei die feste Komplexverbindung durch stöchiometrische Ladungspaarung gebildet wird, wobei der Polyelektrolyt bei einem pH-Wert von 7,4 stabil und bei einem pH-Wert unter 7 abbaubar ist.

14. Verwendung nach Anspruch 13,
**dadurch gekennzeichnet, dass** die Polymermatrix vom Typ Poly-α-Hydroxysäure oder Polyanhydrid oder Polycaprolacton, vorzugsweise PLA oder PLAGA ist oder auf Copolymeren mit hydrophilen Segmenten, wie Polyvinylpyrrolidon, Dextran oder Polyethylenoxid und saure Verbindungen erzeugenden Polymersegmenten, wie Polyanhydride oder Poly-α-Hydroxysäuren basiert.

15. Verwendung nach Anspruch 13 oder 14,
**dadurch gekennzeichnet, dass** der Komplexbildner ausgewählt ist aus PMLA, PLCA, PLCAI oder PSA.

## Claims

1. System for the controlled release of an active principle comprising at least (a) one hydrolytically degradable polymer matrix forming acid compounds and (b) at least one complex of an active principle having at least one electrostatic charge and a polyelectrolytic partner which complexes with the active principle, of opposite charge, said complexing agent being acid-sensitive stable at a pH of 7.4 and degradable at a pH less than 7, the solid complex being formed by means of stoichiometric pairing of charges.

2. System according to claim 1, **characterised in that** the polymer matrix is of the poly(α-hydroxy acid) or polyanhydride or polycaprolactone type.

3. System according to claim 1 or 2, **characterised in that** the polymer matrix is of the poly(lactide-coglycolide) type.

4. System according to any of claims 1 to 3, **characterised in that** the degradable polymer matrix is based on copolymers comprising hydrophilic segments such as poly(vinyl pyrrolidone), dextran or poly(ethylene oxide) and polymer segments forming acid compounds such as polyanhydrides or poly(α-hydroxy acids).

5. System according to any of claims 1 to 4, **characterised in that** the complexing agent is selected from polyelectrolyte complexing agents of the poly(malic acid) (PMLA), poly(lysine citramide) (PLCA or PLCAI) and poly(amino serinate) (PSA) type.

6. System according to any of claims 1 to 5, **characterised in that** the complex is formed by means of ionic pairing between a basic active principle and a poly(malic acid) or poly(lysine citramide) type polyanion complexing agent.

7. System according to any of claims 1 to 6, **characterised in that** the basic active principle is of the multiamine type.

8. System according to any of claims 1 to 5, **characterised in that** the acid-sensitive complex is formed by means of ionic pairing between an acidic active principle and an acid-sensitive poly(amino serinate) (PSA) type polycation complexing agent.

9. System according to any of claims 1 to 8, **characterised in that** the complex is insoluble in aqueous medium.

10. System according to any of claims 1 to 9, **characterised in that** the active principle is a polyelectrolyte.

11. System according to any of claims 1 to 10, **characterised in that** the ratio of complex in the system is within the range from 0.1 to 60% by weight, preferably 1 to 40% by weight.

12. Method for preparing a controlled release system according to any of claims 1 to 11 comprising at least the steps consisting of forming a complex between an active principle having at least one electrostatic charge and an acid-sensitive complexing polyelectrolyte partner of opposite charge stable at a pH of 7.4 and degradable at a pH less than 7, said complex being stable under the pH, salinity and temperature conditions of physiological media, followed by the incorporation of said complex in a bioabsorbable polymer matrix forming acid hydrolytic degradation products.

13. Use of an acid-sensitive polyelectrolyte as a complexing agent, in a system for the controlled release of an active principle comprising at least one degradable polymer matrix forming acid compounds and at least one complex between an active principle having at least one electrostatic charge and a polyelectrolyte partner which complexes with said active principle, of opposite charge, the solid complex being formed by means of stoichiometric pairing of charges, the polyelectrolyte being stable at a pH of 7.4 and degradable at a pH less than 7.

14. Use according to claim 13, **characterised in that** the polymer matrix is of the poly(α-hydroxy acid) or polyanhydride or polycaprolactone type, preferably, PLA or PLAGA, or is based on copolymers comprising hydrophilic segments such as poly(vinyl pyrrolidone), dextran or poly(ethylene oxide) and polymer segments forming acid compounds such as polyanhydrides or poly(α-hydroxy acids).

15. Use according to claim 13 or 14, **characterised in that** the complexing agent is selected from PMLA, PLCA, PLCAI or PSA.
